# EUROPÄISCHE PATENTSCHRIFT

(11) **EP 0 793 515 B1**
(45) Veröffentlichungstag und Bekanntmachung des Hinweises auf die Patenterteilung: **25.03.1998**
(21) Anmeldenummer: 95935931.6
(22) Anmeldetag: 11.10.1995
(51) Int. Cl.: A61M 21/00

(54) **GERÄT ZUR AKUSTISCHEN BEEINFLUSSUNG PHYSISCHER UND PSYCHISCHER FUNKTIONEN**
DEVICE FOR ACOUSTICALLY AFFECTING PHYSICAL AND PSYCHIC FUNCTIONS
DISPOSITIF PERMETTANT D'INFLUER ACOUSTIQUEMENT SUR DES FONCTIONS PHYSIQUES ET PSYCHIQUES

(30) Priorität: 24.11.1994 DE 9418874 U
(43) Veröffentlichungstag der Anmeldung: 10.09.1997
(73) Patentinhaber: HENNL, Wilhelm, D-86609 Donauwörth (DE)
(72) Erfinder: HENNL, Wilhelm, D-86609 Donauwörth (DE)
(74) Vertreter: Schoppe, Fritz, Dipl.-Ing.
(86) Internationale Anmeldenummer: EP9504009
(87) Internationale Veröffentlichungsnummer: WO9615823

(56) Entgegenhaltungen:
- EP-A- 0 166 011
- WO-A-88/04948
- GB-A- 2 161 974
- US-A- 4 632 126
- US-A- 5 027 686
- US-A- 5 243 998
- US-A- 5 253 168
- PATENT ABSTRACTS OF JAPAN vol. 13 no. 79 (C-571) ,22.Februar 1989 & JP,A,63 267368 (TAKATOSHI ENDO) 4.November 1988,

## Beschreibung

Die vorliegende Erfindung bezieht sich auf ein Gerät zur akustischen Beeinflussung physischer und psychischer Funktionen.

Akustische Signale (Musik, Geräusche u.a.) vermitteln uns nicht nur bewußte Informationen. Sie können ohne den Umweg über unser Bewußtsein auch physische und psychische Funktionen, beispielweise Muskelreaktionen, Herzaktivität, Herzrhythmus, Atmung, Gehirnaktivität, Stimmungen u.a. beeinflussen. Die Funktionen können auf diese Weise wirkungsvoll und nachhaltig verändert werden.

Die alltägliche Musik kann beispielsweise unsere Beine ohne unser bewußtes Zutun im Rhythmus der Musik reagieren lassen; sie kann uns begeistern, bedrücken oder Veränderungen im Herz-Kreislauf-Bereich auslösen.

Geräusche können uns beispielsweise eine "Gänsehaut" über den Rücken laufen lassen, und dies bereits in Bruchteilen einer Sekunde, und ohne daß wir uns dagegen zu wehren vermögen. Geräusche können uns erschauern und frösteln lassen oder Sicherheit, Geborgenheit, freies Atmen und spürbare Lockerheit vermitteln. Durch Musik ausgelöste Veränderungen physischer und psychischer Funktionen sind beispielsweise in dem Buch von Gerhart Harrer "Grundlagen der Musiktherapie und Musikpsychologie" erschienen 1982 im G. Fischer Verlag Stuttgart beschrieben und durch Messungen belegt.

Die Bedeutung von Musik für die Therapie ist bekannt. Beispielsweise wird verwiesen auf das zuvor genannte Buch, weiterhin auf das Manuskript E. Kühn "Medizin mit gutem Klang", Manuskript des Bayerischen Rundfunks zu der Sendung, Sonntag, 26. Oktober 1986, Bayern 2, 20.00 bis 21.00 Uhr. Aus diesen Unterlagen ist es zu entnehmen, daß akustische Reize zur direkten Beeinflussung des vegetativen Nervensystems eingesetzt werden können. Durch geeignete Wahl der akustischen Reize kann auch beispielsweise die Herzfrequenz eines Patienten gesteigert oder reduziert werden.

Weiterhin ist diesem Buch zu entnehmen, daß mit akustischen Therapien beispielsweise bei schweren Schädelhirntraumatas Heilungserfolge erzielt werden können, die mit anderen Mitteln nicht möglich waren. Dies mag als Hinweis auf Einfluß und Möglichkeiten akustischer Einwirkungen gelten.

Nach aktuellem Kenntnisstand sind im alltäglichen Sprachgebrauch als "Geräusche" oder "Geräuschfolgen" bezeichnete akustische Signale ebenfalls für die Therapie geeignet und können gegenüber der Anwendung von "Musik" Vorteile bieten.

So konnte beispielsweise durch bestimmte Geräusche und Anwendungsmechanismen aus einer spürbar überkontrollierten, beklemmend starren Herz-, Kreislauf- und Atmungssituation heraus ein gefühlsmäßig völlig unkontrolliert und frei variierender Herzrhythmus, eine gefühlsmäßig völlig unkontrollierte und frei variierende Herzaktivität bzw. ein derartiges Kreislauf- und Atmungsverhalten erreicht werden.

Nach aktueller wisssenschatlicher Erkenntnis "ist aber gerade diese offenbar unvorhersehbar variierende Herzfrequenz das Kennzeichen des gesunden Herzens, während das kranke Herz dagegen unverändert und starr in einem präzisen Rhythmus schlägt". (Laut dem Bericht "Rhythmus", erschienen in der Zeitschrift "TV Hören und Sehen" Hr. 21/1994, Seiten 4 und 5).

Weiterhin wird verwiesen auf den Bericht vom 12. Weltkongress der Kardiologen (Süddeutsche Zeitung Nr. 219 vom 22.9.1994, Seite 39), nach dem die bisherige Betrachtungsweise bestimmter Herzerkrankungen und Therapien durchaus in Frage zu stellen ist.

Die WO-A-8804948 offenbart ein System nach dem Oberbegriff des Patentanspruchs 1, bei dem mittels eines Sensorsystems die Position und/oder Bewegungen von Künstlern auf einer Bühne erfaßt werden, wobei auf der Basis der erfaßten Position und/oder Bewegung visuelle oder akustische Effekte bewirkt werden. Beispielsweise wird auf der Grundlage von Ausgangssignalen des Sensorsystems die Lautstärke oder die Tonhöhe einer akustischen Ausgabe beeinflußt. Die WO-A-8804948 befaßt sich jedoch nicht mit einem Gerät zur akustischen Beeinflussung physischer und psychischer Funktionen eines Patienten.

Im Hinblick auf diesen Stand der Technik liegt der vorliegenden Erfindung die Aufgabe zugrunde, ein Gerät zu schaffen, das durch bestimte Steuersignale akustische Signale zur Beeinflussung physischer und psychischer Funktionen erzeugt.

Diese Aufgabe wird durch ein Gerät zur akustischen Beeinflussung physischer und psychischer Funktionen gemäß Patentanspruch 1 gelöst.

Die vorliegende Erfindung schafft ein Gerät zur akustischen Beeinflussung physischer und psychischer Funktionen, die eine Steuersignalerzeugungseinrichtung, die Sensoren zur Erfassung der Bewegung eines Patienten umfaßt, die räumlich entfernt von dem Patienten anortenbar sind, und eine Gerauscherzeugungseinrichtung, deren Eingang mit einem Ausgang der Steuersignalerzeugungseinrichtung verbunden ist, die abhängig von den empfangenen Signalen ein Geräusch erzeugt und ausgibt, wobei für jedes Geräusch dessen Schwebungsfrequenz und/oder dessen Oberwellengehalt und/oder dessen Hüllkurve abhängig von den empfangenen Signalen festgelegt ist, aufweist.

Das erfindungsgemäße Gerät schafft eine durch eine Mehrzahl von Steuersignalen wählbare Klangumgebung des Patienten.

Die vorliegende Erfindung stellt eine "sanfte Technologie" dar. Sie bietet verbesserte Therapiemöglichkeiten bei Erkrankungen, für die bislang keine effektiven Therapien vorhanden waren. Nachteilige Nebenwirkungen konnten nicht beobachtet werden.

Die für die jeweiligen Situationen relevanten akustischen und physischen/psychischen Phänomene und Hypothesen seien an dieser Stelle nicht betrachtet. Es sei jedoch darauf hingewiesen, daß sich damit auch das Zustandekommen der "beklemmend starren Herz-, Kreislauf und Atmungssituation", die im zuvor angeführten Beispiel Ausgangszustand war, erklären läßt.

Der Erfindung liegt die Erkenntnis zugrunde, daß einem Patienten eine für ihn entspannend wirkende akustische Umgebung dadurch geboten werden kann, daß ein vom Zustand des Patienten oder dessen Umgebung abhängiges Steuersignal durch die Steuersignalerzeugungseinrichtung erzeugt wird und abhängig von diesen Steuersignalen mittels der Geräuscherzeugungseinrichtung ein akustisches Geräusch erzeugt wird.

Die Erfindung bewirkt demgemäß eine akustisch und/oder rhythmisch interessante Umgebung mit einem nach dem jeweiligen Zustand und Verhalten des Patienten differenzierten Klangverhalten, Rhythmus- oder Geräuschbild.

Weiterbildungen des erfindungsgemäßen Geräts zur akustischen Beeinflussung phyischer und psychischer Funktionen sind in den Unteransprüchen definiert.

Nachfolgend werden unter Bezugnahme auf die beiliegenden Zeichnungen bevorzugte Ausführungsbeispiele des erfindungsgemäßen Geräts näher erläutert. Es zeigen:
- Fig. 1: ein Blockdiagramm eines ersten Ausführungsbeispiels der vorliegenden Erfindung;
- Fig. 2: ein Blockdiagramm eines zweiten Ausführungsbeispiels der vorliegenden Erfindung; und
- Fig. 3: eine detaillierte Darstellung der Steuersignalerzeugungseinrichtung.

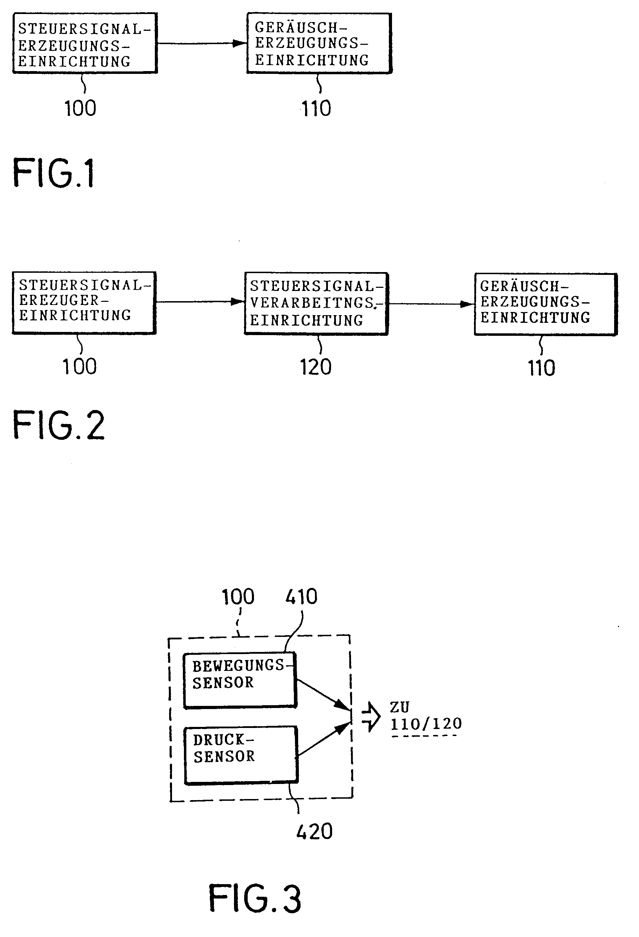

Wie in Fig. 1 zu sehen ist, umfaßt das erfindungsgemäße Gerät zur akustischen Beeinflussung physischer und psychischer Funktionen eine Steuersignalerzeugungseinrichtung 100 und eine Geräuscherzeugungseinrichtung 110. Der Ausgang der Steuersignalerzeugungseinrichtung 100 ist mit dem Eingang der Geräuscherzeugungseinrichtung 110 verbunden. Die Geräuscherzeugungseinrichtung 110 empfängt die Steuersignale von der Steuersignalerzeugungseinrichtung 100 und erzeugt abhängig von den empfangenen Signalen ein akustisches Geräusch und gibt dieses aus. Abhängig von den empfangenen Steuersignalen ist für jedes Geräusch dessen Tonhöhe der Grundfrequenz und/oder der Schwebungsfrequenz oder dessen Grundrhythmus und/oder dessen Oberwellengehalt und/oder dessen Hüllkürve festgelegt.

In Fig. 2 ist ein zweites Ausführungsbeispiel des erfindungsgemäßen Geräts dargestellt. Neben den bereits in Fig. 1 beschriebenen Elementen, nämlich der Steuersignalerzeugungseinrichtung 100 und der Geräuscherzeugungseinrichtung 110 umfaßt dieses zweite Ausführungsbeispiel eine Steuersignalverarbeitungseinrichtung 120. Die Steuersignalverarbeitungseinrichtung 120 ist zwischen dem Ausgang der Steuersignalerzeugungseinrichtung 100 und dem Eingang der Geräuscherzeugungseinrichtung 110 angeordnet. Die Steuersignalverarbeitungseinrichtung 120 verarbeitet abhängig von vorgegebenen Einstellungen die von der Steuersignalerzeugungseinrichtung empfangenen Steuersignale und gibt die verarbeiteten Signale an die Geräuscherzeugungseinrichtung 110 ab.

Die Einstellungen der Steuersignalverarbeitungseinrichtung können beispielsweise vorbestimmte Parameter umfassen, die in einer Speichervorrichtung (nicht dargestellt) der Steuersignalverarbeitungseinrichtung 120 gespeichert sind und anhand derer die empfangenen Steuersignale verändert, abgestimmt oder optimiert werden, um so mittels der Geräuscherzeugungseinrichtung 110 vorbestimmte akustische Signale zu erzeugen.

In Fig. 3 ist die Steuersignalerzeugungseinrichtung 100 gemäß der vorliegenden Erfindung gezeigt, bei der Sensoren verwendet werden 410, 420, die vom Patienten entfernt angeordnet sind. Bei diesen Sensoren handelt es sich um einen Bewegungssensor 410 und einen Drucksensor 420. Diese Sensoren können beispielsweise in einem Krankenzimmer angeordnet sein, um dadurch die Bewegung und den Bewegungsumfang eines Patienten zu erfassen.

Die durch die Sensoren 410, 420 ausgegebenen Steuersignale werden entweder einzeln an die Geräuscherzeugungseinrichtung 110 oder an die Steuersignalverarbeitungseinrichtung 120 abgegeben oder zu einem Signal zusammengefaßt und gemeinsam an diese übertragen.

In Abweichung von den oben beschriebenen Ausführungsbeispielen sind weitere Formen der Steuersignalerzeugungseinrichtung 100 möglich.

Unter dem Begriff "Geräusch" im Sinne der vorliegenden Anmeldung sollen alle Geräuscharten, Klänge und Rhythmen verstanden werden.

## Patentansprüche

1. Gerät zur akustischen Beeinflussung physischer und psychischer Funktionen, mit
- einer Steuersignalerzeugungseinrichtung (100), die Sensoren (410, 420) zur Erfassung der Bewegung eines Patienten umfaßt, die räumlich entfernt von dem Patienten anortenbar sind; und
- einer Geräuscherzeugungseinrichtung (110), deren Eingang mit einem Ausgang der Steuersignalerzeugungseinrichtung (100) verbunden ist, die abhängig von den empfangenen Signalen ein Geräusch erzeugt und ausgibt,
dadurch gekennzeichnet, daß für jedes Geräusch dessen Schwebungsfrequenz und/oder dessen Oberwellengehalt und/oder dessen Hüllkurve abhängig von den empfangenen Signalen festgelegt ist.

2. Gerät nach Anspruch 1, dadurch gekennzeichnet,
daß zwischen dem Ausgang der Steuersignalerzeugungseinrichtung (100) und dem Eingang der Geräuscherzeugungseinrichtung (110) eine Steuersignalverarbeitungseinrichtung (120) angeordnet ist, die von der Steuersignalerzeugungseinrichtung (100) empfangene Steuersignale verarbeitet und an die Geräuscherzeugungseinrichtung (110) abgibt.

## Claims

1. A device for acoustically affecting physical and psychic functions comprising
- a control signal generating unit (100), which includes sensors (410, 420) for registering the movement of a patient which can be positioned at a distance from the patient; and
- a noise generating unit (110) whose input is connected to an output of the control signal generating unit (100) and which generates and outputs a noise depending on the received signals,
characterized in that the vibrational frequency and/or the harmonic content and/or the envelope curve for each noise is determined depending on the received signals.

2. A device according to claim 1, characterized in
that a control signal processing unit (120) which processes the control signals received from the control signal generating unit (100) and delivers them to the noise generating unit (110) is arranged between the output of the control signal generating unit (100) and the input of the noise generating unit (110).

## Revendications

1. Dispositif destiné à influencer des fonctions physiques et psychiques par des effets acoustiques, incluant:
un appareil générateur (100) de signaux de commande, qui comprend des capteurs (410, 420) de détection du mouvement d'un patient qui peuvent être positionnés dans l'espace à distance du patient ; et
un appareil générateur (110) de bruit, dont l'entrée est reliée à une sortie de l'appareil générateur (100) de signaux de commande et qui engendre et émet un bruit en fonction des signaux reçus,
caractérisé en ce que la fréquence de battement et/ou la teneur en harmoniques et/ou l'enveloppante est définie, pour chaque bruit, en fonction des signaux reçus.

2. Dispositif selon la revendication 1, caractérisé en ce que
un appareil de traitement (120) de signaux de commande, qui traite des signaux de commande reçus de l'appareil générateur de signaux de commande et les envoie à l'appareil générateur (110) de bruit, est agencé entre la sortie de l'appareil générateur (100) de signaux de commande et l'entrée de l'appareil générateur (110) de bruit.
